(19) Europäisches Patentamt
European Patent Office
Office européen des brevets

(11) **EP 1 471 061 B1**

(12) **EUROPEAN PATENT SPECIFICATION**

(45) Date of publication and mention
of the grant of the patent:
**27.02.2008 Bulletin 2008/09**

(51) Int Cl.:
**C07D 301/19** (2006.01)    **C07D 303/04** (2006.01)

(21) Application number: **02790879.7**

(86) International application number:
**PCT/JP2002/013566**

(22) Date of filing: **26.12.2002**

(87) International publication number:
**WO 2003/057682 (17.07.2003 Gazette 2003/29)**

(54) **PROCESS FOR PRODUCTION OF PROPYLENE OXIDE**

VERFAHREN ZUR HERSTELLUNG VON PROPYLENOXID

PROCEDE DE PRODUCTION D'OXYDE DE PROPYLENE

(84) Designated Contracting States:
**AT BE BG CH CY CZ DE DK EE ES FI FR GB GR
IE IT LI LU MC NL PT SE SI SK TR**

(30) Priority: **08.01.2002   JP 2002001164**

(43) Date of publication of application:
**27.10.2004   Bulletin 2004/44**

(73) Proprietor: **Sumitomo Chemical Company,
Limited
Tokyo 104-8260 (JP)**

(72) Inventor: **TSUJI, Junpei
Ichihara-shi, Chiba 299-0125 (JP)**

(74) Representative: **Henkel, Feiler & Hänzel
Patentanwälte
Maximiliansplatz 21
80333 München (DE)**

(56) References cited:
**WO-A-01/70711          WO-A-01/70714
JP-A- 2001 031 662     JP-A- 2001 270 873
JP-A- 2001 270 878**

• **PATENT ABSTRACTS OF JAPAN vol. 2002, no.
02, 2 April 2002 (2002-04-02) & JP 2001 270880 A
(SUMITOMO CHEM CO LTD), 2 October 2001
(2001-10-02)**
• **PATENT ABSTRACTS OF JAPAN vol. 2002, no.
02, 2 April 2002 (2002-04-02) & JP 2001 270875 A
(SUMITOMO CHEM CO LTD), 2 October 2001
(2001-10-02)**

## Description

Technical Field

The present invention relates to a process for producing propylene oxide. More particularly, the present invention relates to a process for producing propylene oxide, having excellent characteristics that propylene can be converted into desired propylene oxide by epoxidation without production of styrene as a by-product, that cumene constituting a carrier of oxygen used in epoxidation, can be repeatedly used, and further that deterioration of an activity caused by poisoning of a solid catalyst used in a hydrogenolysis step, can be prevented and the catalyst life can be also maintained for long time.

Background Art

A process for oxidizing propylene by using a hydroperoxide of ethylbenzene as an oxygen carrier to obtain propylene oxide and styrene, is known as a Halcon process. This process , however, is unsatisfactory from the standpoint of obtaining selectively only propylene oxide because styrene as a by-product is necessarily produced together with propylene oxide.

JP-A-2001-031662 discloses a method for producing propylene oxide which comprises the following processes:

the oxidation process: a process for oxidizing isopropylbenzene to obtain isopropylbenzene hydroperoxide;

the epoxidation process: a process for reacting the obtained isopropylbenzene hydroperoxide with propylene to obtain the propylene oxide and cumyl alcohol;

the hydrogenolysis process: a process for the hydrogenolysis of the obtained cumyl alcohol to obtain isopropylbenzene which is then recycled as a raw material for the oxidation process;

the organic acid-removing process: a process for removing organic acids outside the system in the above-mentioned processes, respectively, or in at least one of lines for binding the processes.

Disclosure of the Invention

Objects of the present inventors are to provide a process for producing propylene oxide, having excellent characteristics that propylene can be converted into desired propylene oxide by epoxidation without production of styrene as a by-product, that cumene constituting a carrier of oxygen used in epoxidation, can be repeatedly used, and further that deterioration of the activity caused by poisoning of a solid catalyst used in a hydrogenolysis step, can be prevented and the catalyst life can be also maintained for long time.

Namely, the present invention relates to a process for producing propylene oxide comprising the following steps:

oxidation step: a step of obtaining cumene hydroperoxide by oxidizing cumene;
epoxidation step: a step of obtaining propylene oxide and cumyl alcohol by reacting a cumene solution containing cumene hydroperoxide obtained in the oxidation step with an excess of propylene in the presence of a solid catalyst in a liquid phase; and
hydrogenolysis step: a step of obtaining cumene by subjecting cumyl alcohol obtained in the epoxidation step to hydrogenolysis, and recycling the cumene to the oxidation step as the raw material in the oxidation step,
wherein the concentration of organic acids having a carboxyl group in cumyl alcohol supplied to the hydrogenolysis step is adjusted to 200 ppm by weight or less.

Best Mode for Carrying out the Invention

The oxidation step is a step for obtaining cumene hydroperoxide by oxidizing cumene. The oxidation of cumene is usually conducted by auto-oxidation using an oxygen-containing gas such as air, oxygen-concentrated air or the like. This oxidation may be conducted without use of an additive, and an additive such as an alkali may be used. The reaction temperature is usually from 50 to 200˚C, and the reaction pressure is usually between atmospheric pressure and 5 MPa. In the oxidation method in which the additive is used, an alkali metal compound such as NaOH or KOH, an alkaline earth metal compound, or alkali metal carbonate such as $Na_2CO_3$ or $NaHCO_3$, ammonia, $(NH_4)_2CO_3$, alkali metal ammonium carbonates or the like, is used as the alkali.

The epoxidation step is a step for obtaining propylene oxide and cumyl alcohol by reacting cumene hydroperoxide obtained in the oxidation step with propylene in the presence of a solid catalyst in a liquid phase.

As the catalyst, catalysts containing titanium-containing silicon oxide, are preferable from the viewpoint of obtaining the objective product under high yield and high selectivity.

As these catalysts, so-called Ti-silica catalysts containing Ti chemically bonded to silicon oxide, are preferable. For example, a catalyst prepared by supporting a Ti compound on a silica carrier, a catalyst prepared by combining a Ti compound with silicon oxide by a coprecipitation method or sol gel method, zeolite compounds containing Ti, and the like, can be listed.

In the present invention, cumene hydroperoxide used as the raw material for the epoxidation process, may be a dilute or dense purified material or non-purified material.

The epoxidation is conducted by contacting propylene and cumene hydroperoxide with a catalyst. The reaction is carried out in a liquid phase using a solvent. The solvent must be liquid under temperature and pressure in the reaction, and substantially inert to reactants and products. The solvent may be that which is composed of a material present in a hydroperoxide solution used. For example, when cumene hydroperoxide is a mixture with cumene which is a raw material thereof, the cumene can be used instead of a solvent without particularly adding a solvent. Additionally, monocyclic aromatic compounds (e.g. benzene, toluene, chlorobenzene, orthodichlorobenzene), and alkanes (e.g. octane, decane, dodecane) and the like are listed as useful solvents.

The epoxidation temperature is usually from 0 to 200˚C, and preferably from 25 to 200˚C. The pressure may be at a level sufficient to keep the reaction mixture in a liquid condition. In general, the pressure is advantageously from 100 to 10000 kPa.

The solid catalyst can be advantageously used in the form of a slurry or fixed bed. In the case of a large-scale industrial operation, a fixed bed is preferably used. The epoxidation can be conducted by a batch-wise method, semi-continuous method, continuous method or the like. When a liquid containing raw materials for the reaction is passed through a fixed bed, a liquid-like mixture discharged from a reaction zone does not contain the catalyst at all or substantially.

In the present invention, it is necessary to adjust the concentration of organic acids in cumyl alcohol supplied to the hydrogenolysis step to 200 ppm by weight or less, preferably 50 ppm or less. When the total concentration of the above-described organic acids exceeds the above-described range, the effect of the present invention described before cannot be realized. Besides, the total concentration of the organic acids can be determined by an ion chromatography, gas chromatography or the like. The organic acids are those having a carboxyl group and include carboxylic acids such as formic acid, acetic acid, propionic acid and benzoic acid, dicarboxylic acids such as oxalic acid and malonic acid, hydroxy acids such as lactic acid, and the like.

As methods of controlling the concentration of the organic acids contained in cumyl alcohol supplied to the hydrogenolysis step to the above-described range, distillation, extraction using water or an alkaline aqueous solution, or the like can be conducted. The alkaline aqueous solution similar to those described above, can be used, but aqueous solutions of hydroxides of alkali metals or hydroxides alkaline earth metals, are preferred. The organic acids are generated by decomposition of the organic peroxide between the oxidation step and the epoxidation step, as a main cause.

The hydrogenolysis step in the present invention is a step in which cumyl alcohol obtained in the epoxidation step is subjected to hydrogenolysis to obtain cumene and cumene is recycled to the oxidation step as the raw material of the oxidation step. Namely, by hydrogenolysis, that which is the same as cumene used in the oxidation step is reproduced. The hydrogenolysis reaction is usually conducted by contacting cumyl alcohol and hydrogen with a catalyst. The reaction can be carried out in a liquid phase using a solvent, or in gas phase. The solvent must be substantially inert to reactants and products. The solvent may be that which is a material present in a cumyl alcohol solution used. For example, when cumyl alcohol is a mixture with cumene which is a product, the cumene can be used instead of a solvent without particularly adding a solvent. Additionally, alkanes (e.g., octane, decane, dodecane), monocyclic aromatic compounds (e.g., benzene, ethylbenzene, toluene) and the like are listed as useful solvents. The hydrogenolysis temperature is generally from 0 to 500˚C, and preferably from 30 to 400˚C. In general, the pressure is advantageously from 100 to 10000 kPa. The hydrogenolysis can be advantageously conducted using a catalyst in the form of a slurry or fixed bed. Any catalyst having a hydrogenation ability can be used as the catalyst. Examples of the catalyst include metal-based catalysts of metals of the group 8th to 10th such as cobalt, nickel and palladium, and metal-based catalysts of metals of the group 11th or 12th such as copper and zinc, and copper-based catalysts are preferred from the viewpoint that by-products are suppressed. The copper-based catalysts include copper, Raney copper, copper-chromium, copper-zinc, copper-chromium-zinc, copper-silica, copper-alumina and compounds containing these. The method of the present invention can be conducted by a batch-wise method, semi-continuous method or continuous method. When liquid or gas containing raw materials for the reaction is passed through a fixed bed, a liquid-like mixture discharged from a reaction region does not contain the catalyst at all or substantially.

Example

The present invention will be illustrated by using Examples below.

Example 1

A cumene solution containing 25% by weight of cumyl alcohol was passed together with hydrogen through a reactor in which a copper-chromium catalyst was packed to react them. In this case, the total concentration of formic acid and acetic acid in the cumene solution was 10 ppm by weight. A molar ratio of hydrogen to cumyl alcohol of 8, LHSV of cumene of 1.5 hour$^{-1}$, and reaction pressure of 1 MPa-G(gauge pressure) were adopted. Only cumene was produced in the reaction of cumyl alcohol. The result is shown in Table 1.

Example 2

It was carried out in the same manner as in Example 1 except that the total concentration of formic acid and acetic acid in the cumene solution was 40 ppm by weight. The result is shown in Table 1.

Example 3

It was carried out in the same manner as in Example 1 except that the total concentration of formic acid and acetic acid in the cumene solution was 100 ppm by weight. The result is shown in Table 1.

Comparative Example 1

It was carried out in the same manner as in Example 1 except that the total concentration of formic acid and acetic acid in the cumene solution was 300 ppm by weight. The result is shown in Table 1.

Table 1

| | Example 1 | Example 2 | Example 3 | Comparative Example 1 |
|---|---|---|---|---|
| Organic acid concentration (ppm by weight) | 10 | 40 | 100 | 300 |
| CMA conversion* (%) | 99.9 | 99.8 | 99.5 | 98.0 |

\* CMA conversion (%) = [(cumyl alcohol concentration in raw material – cumyl alcohol concentration in reacted solution) /cumyl alcohol concentration in raw material] x 100

Industrial Applicability

As described above, according to the present invention, there can be provided a process for producing propylene oxide, having excellent characteristics that propylene can be converted into propylene oxide by epoxidation without production of styrene as a by-product, that cumene constituting a carrier of oxygen used in epoxidation, can be repeatedly used, and further that deterioration of an activity caused by poisoning of a catalyst used in a hydrogenolysis step, can be prevented and the catalyst life can be also maintained for long time.

**Claims**

1.    A process for producing propylene oxide, comprising the following steps:

oxidation step: a step of obtaining cumene hydroperoxide by oxidizing cumene;
epoxidation step: a step of obtaining propylene oxide and cumyl alcohol by reacting a cumene solution containing cumene hydroperoxide with an excess of propylene in a liquid phase in the presence of a solid catalyst; and
hydrogenolysis step: a step of obtaining cumene through hydrogenolysis of cumyl alcohol obtained in the epoxidation step, and recycling the cumene to the oxidation step as the raw material of the oxidation step,
wherein the concentration of organic acids having a carboxyl group in cumyl alcohol supplied to the hydrogenolysis step is adjusted to 200 ppm by weight or less.

2.    The process according to claim 1, wherein the concentration of the organic acids having a carboxyl group in cumyl alcohol is adjusted to 50 ppm by weight or less.

3.    The process according to claim 1, wherein the solid catalyst of the hydrogenolysis step is a copper-based catalyst.

4.    The process according to claim 2, wherein the solid catalyst of the hydrogenolysis step is a copper-based catalyst.

**Patentansprüche**

1.    Verfahren zur Herstellung von Propylenoxid, das die folgenden Stufen umfasst:

Oxidationsstufe: eine Stufe der Gewinnung von Cumolhydroperoxid durch Oxidation von Cumol;
Epoxidationsstufe: eine Stufe der Gewinnung von Propylenoxid und Cumylalkohol durch Umsetzung einer Cumolhydroperoxid enthaltenden Cumollösung mit einem Überschuss von Propylen in einer flüssigen Phase in Gegenwart eines festen Katalysators; und
Hydrogenolysestufe: eine Stufe der Gewinnung von Cumol durch Hydrogenolyse des in der Epoxidationsstufe erhaltenen Cumylalkohols und der Rückführung des Cumols in die Oxidationsstufe als Ausgangsmaterial für die Oxidationsstufe,
wobei die Konzentration von eine Carboxylgruppe aufweisenden organischen Säuren in dem der Hydrogenolysestufe zugeführten Cumylalkohol auf 200 ppm, bezogen auf das Gewicht, oder weniger eingestellt ist.

2.    Verfahren nach Anspruch 1, wobei die Konzentration der eine Carboxylgruppe aufweisenden organischen Säuren in dem Cumylalkohol auf 50 ppm, bezogen auf das Gewicht, oder weniger eingestellt ist.

3.    Verfahren nach Anspruch 1, wobei der feste Katalysator der Hydrogenolysestufe ein Katalysator auf Kupferbasis ist.

4.    Verfahren nach Anspruch 2, wobei der feste Katalysator der Hydrogenolysestufe ein Katalysator auf Kupferbasis ist.

**Revendications**

1.    Procédé pour produire de l'oxyde de propylène, comprenant les étapes suivantes :

étape d'oxydation : une étape consistant à obtenir de l'hydroperoxyde de cumène en oxydant du cumène ;
étape d'époxydation : une étape consistant à obtenir de l'oxyde de propylène et de l'alcool cumylique en faisant réagir une solution de cumène contenant de l'hydroperoxyde de cumène avec un excès de propylène dans une phase liquide en présence d'un catalyseur solide ; et

étape d'hydrogénolyse : une étape consistant à obtenir du cumène par hydrogénolyse de l'alcool cumylique obtenu dans l'étape d'époxydation, et à recycler le cumène à l'étape d'oxydation en tant que matière première de l'étape d'oxydation,

dans lequel la concentration d'acides organiques ayant un groupe carboxyle dans l'alcool cumylique apporté à l'étape d'hydrogénolyse est réglée à 200 ppm en poids ou moins.

2. Procédé selon la revendication 1, dans lequel la concentration des acides organiques ayant un groupe carboxyle dans l'alcool cumylique est réglée à 50 ppm en poids ou moins.

3. Procédé selon la revendication 1, dans lequel le catalyseur solide de l'étape d'hydrogénolyse est un catalyseur à base de cuivre.

4. Procédé selon la revendication 2, dans lequel le catalyseur solide de l'étape d'hydrogénolyse est un catalyseur à base de cuivre.

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2001031662 A